# EUROPÄISCHE PATENTSCHRIFT

(11) **EP 1 663 352 B1**
(45) Veröffentlichungstag und Bekanntmachung des Hinweises auf die Patenterteilung: **11.07.2007**
(21) Anmeldenummer: 04761891.3
(22) Anmeldetag: 01.09.2004
(51) Int. Cl.: A61M 5/20

(54) **SYSTEM ZUR VERABREICHUNG EINES INJIZIERBAREN PRODUKTS**
SYSTEM FOR ADMINISTERING AN INJECTABLE PRODUCT
SYSTEME POUR ADMINISTRER UN PRODUIT INJECTABLE

(30) Priorität: 03.09.2003 DE 10340584
(43) Veröffentlichungstag der Anmeldung: 07.06.2006
(73) Patentinhaber: TecPharma Licensing AG, 3401 Burgdorf (CH)
(72) Erfinder: HOMMANN, Edgar, CH-3257 Grosshoechstetten (CH); RINDLISBACHER, Christoph, CH-3067 Boll (CH); SCHERER, Benjamin, CH-8610 Uster (CH); THOMPSON, Ian, CH-3400 Burgdorf (CH)
(86) Internationale Anmeldenummer: PCT/CH2004/000551
(87) Internationale Veröffentlichungsnummer: WO 2005/021071

(56) Entgegenhaltungen:
- WO-A-00/18454
- WO-A-01/17593
- WO-A-01/49348
- GB-A- 1 263 355
- US-A- 4 955 870
- US-A- 5 695 472

## Beschreibung

Die vorliegende Erfindung betrifft ein System zur Verabreichung eines injizierbaren Produkts, das eine Injektionsspritze, eine Antriebseinheit und ein Gehäuse zur Aufnahme der Injektionsspritze und der Antriebseinheit umfasst.

Bei verschiedenen medizinischen und therapeutischen Anwendungen ist die Verabreichung eines injizierbaren Produkts erforderlich. Die einfachste Möglichkeit zur Verabreichung des Produkts ist die Verwendung einer herkömmlichen Injektionsspritze. Das Produkt ist bereits in einer Kammer der Spritze aufgenommen oder muss erst in diese aufgezogen werden. Anschließend wird eine an der Injektionsspritze festsitzende Injektionsnadel der Injektionsspritze von Hand in ein Körpergewebe eingestochen und das Produkt durch Vorschub eines Vorschubgliedes, wie etwa einer Kolbenstange, von Hand aus der Injektionsspritze verabreicht. Die Verabreichung des Produkts mit einer Injektionsspritze bedarf daher einer gewissen Übung, damit das Einstechen der Nadel nicht schmerzhaft ist und das Produkt mit einer erforderlichen Geschwindigkeit und in gewünschtem Umfang verabreicht wird. Weiter entsteht durch die Verwendung einer Injektionsnadel eine Verletzungsgefahr durch die offenliegende Nadel.

Zur Vereinfachung der Verabreichung eines injizierbaren Produkts, so dass z. B. auch ein Leihe oder ein Patient selbst eine Verabreichung durchführen kann, wurden verschiedenartige Verabreichungsvorrichtungen entwickelt. Solche Verabreichungsvorrichtungen weisen im allgemeinen eine Kammer zur Aufnahme des injizierbaren Produkts und eine Verabreichungseinrichtung auf. Das injizierbare Produkt kann unmittelbar von der Kammer aufgenommen werden oder es kann eine Ampulle in die Kammer eingesetzt werden, in der das Produkt untergebracht ist. Die Verabreichungsvorrichtung kann ein Antriebsmittel zum Antrieb eines Vorschubglieds aufweisen, um durch den Vorschub des Vorschubglieds das Produkt aus der Kammer zu verabreichen. Ferner sind Verabreichungsvorrichtungen bekannt, die eine Einstechhilfe zum automatischen Einstechen einer Injektionsnadel aus der Verabreichungsvorrichtung in ein Körpergewebe umfassen.

Außerdem sind verschiedenartige Systeme bekannt, die eine herkömmliche Injektionsspritze und eine Verabreichungsvorrichtung zur automatischen Verabreichung des injizierbaren Produkts aus der Spritze kombinieren. Ferner kann eine Dosiervorrichtung vorgesehen sein, durch die die Abgabe einer voreinstellbaren Dosis aus der Injektionsspritze ermöglicht wird. Durch ein solches System wird die Verabreichung des injizierbaren Produkts aus der Injektionsspritze vereinfacht. Es ist jedoch weiterhin erforderlich, die Injektionsnadel von Hand in das Körpergewebe einzustechen und nach der Verabreichung des Produkts wieder aus diesem herauszuziehen, wobei eine Verletzungsgefahr an der freiliegenden Nadel besteht. Es kann eine Nadelschutzkappe über der Injektionsnadel vorgesehen werden. Diese muss jedoch vor und nach der Verabreichung von Hand über der Nadel angeordnet werden. Diese Handgriffe verzögern den Verabreichungsvorgang eines injizierbaren Produkts und weisen ebenfalls eine Verletzungsgefahr an der Injektionsnadel auf.

Aus US 5,695,472 ist z.B. eine Injektionsanordnung mit einem Medikamentenmodul und einem Betätigungsmodul bekannt, die zusammen eine wiederaufladbare automatische Injektionsvorrichtung bilden. Das Medikamentenmodul besteht aus einem ersten Gehäuseteil, in dem eine Patrone mit einer Injektionsnadel angeordnet ist, wobei die Patrone innerhalb dem Gehäuseteil durch eine Feder in einer eingefahrenen Stellung gehalten wird. Das Betätigungsmodul umfasst ein Gehäuse, in dem ein Federantrieb und eine Auslöseeinrichtung für den Federantrieb vorgesehen sind. Das Gehäuseteil des Medikamentenmoduls und das Gehäuseteils des Antriebsmoduls können miteinander verschraubt werden und bilden so das Injektionsgerät. In der GB 1,263,355 ist ein Injektionsgerät beschrieben, das ebenfalls aus einem Gehäuseteil mit einem Federantrieb und aus einem Gehäuseteil mit einer Injektionsspritze aufgebaut ist. Die Gehäuseteile werden über eine Schraubverbindung miteinander verbunden. Die Injektionsspritze wird in dem Gehäuseteil mittels eines nach innen ragenden Wulstes durch Reibschluss gehalten. Der Wulst schliesst mit dem Aussenumfang der Injektionsspritze den Innenraum des Gehäuses zur einen Seite ab. Zur anderen Seite, durch welche die Injektionsnadel vorgeschoben wird, ist das Gehäuse durch eine Gummiversiegelung abgeschlossen.

Es ist die Aufgabe der vorliegenden Erfindung, ein System zur Verabreichung eines injizierbaren Produkts aus einer Injektionsspritze bereit zu stellen, das eine einfache und zuverlässige Verabreichung des Produkts ermöglicht, die Verletzungsgefahr bei einem Verabreichungsvorgang verringert und kostengünstig ist.

Die Aufgabe wird durch ein System zur Verabreichung eines injizierbaren Produkts nach dem Patentanspruch 1 erfüllt. Vorteilhafte Ausgestaltungen des Systems gehen aus den Unteransprüchen hervor.

Demnach umfasst ein erfindungsgemäßes System zur Verabreichung eines injizierbaren Produkts eine Injektionsspritze, eine Antriebseinheit und ein Gehäuse zur Aufnahme der

Injektionsspritze und der Antriebseinheit. Die Injektionsspritze weist ein Spritzengehäuse auf, in dem eine Produktkammer für das injizierbare Produkt aufgenommen ist. Die Produktkammer ist an einer Seite durch einen relativ zum Spritzengehäuse beweglichen Stopfen abgeschlossen. An das Spritzengehäuse, bzw. die Produktkammer, schließt sich eine Injektionsnadel an, die eine Fluidverbindung zur Produktkammer besitzt. Durch Vorschub eines Vorschubgliedes, wie einer Kolbenstange, die den Stopfen innerhalb der Produktkammer antreibt, kann das Produkt durch die Injektionsnadel aus der Injektionsspritze verabreicht werden. Die Antriebseinheit weist vorzugsweise einen mechanischen Antrieb auf, kann jedoch auch einen anderen Antrieb, wie z. B. einen Gasantrieb, umfassen. Erfindungsgemäß ist die Antriebseinheit derart ausgebildet, dass sie die Injektionsspritze in Längsrichtung des Gehäuses relativ zum Gehäuse antreibt, bis die Injektionsnadel der Injektionsspritze aus dem Gehäuse ragt. Das heißt die Injektionsspritze wird als Einheit von der Antriebseinheit innerhalb dem Gehäuse verschoben, um die Injektionsnadel der Injektionsspritze in ein Körpergewebe einzustechen und dabei vorteilhafter Weise den gesamten erforderlichen Einstechweg zurück zu legen. Vorzugsweise erfolgt die Bewegung der Injektionsspritze in der Vorschubrichtung des Vorschubgliedes der Spritze, das zur Verabreichung des Produkts vorgesehen ist und damit in Verabreichungsrichtung.

Mit Hilfe eines Verabreichungssystems nach der vorliegenden Erfindung ist es bei einer Verabreichung eines injizierbaren Produkts möglich, eine herkömmliche Injektionsspritze zu verwenden, die kostengünstig als Massenware hergestellt werden kann. Ferner kann die Verletzungsgefahr bei der Verwendung einer solchen Injektionsspritze verringert werden, das das Einstechen der Injektionsnadel der Injektionsspritze automatische direkt aus dem Gehäuse des Systems erfolgt, wobei das Gehäuse eine Nadelschutzfunktion übernimmt. Die Nadel kann direkt aus dem Gehäuse in ein Körpergewebe eingestochen werden, wenn das Gehäuse auf eine Gewebeoberfläche aufgesetzt wird, so dass eine Verletzungsgefahr ausgeschlossen werden kann.

Bei dem Verabreichungssystem nach der vorliegenden Erfindung sind die Injektionsspritze, die Antriebseinheit und das Gehäuse vorteilhafterweise als getrennte Bauteile ausgebildet, die nach ihrem Zusammensetzen eine Vorrichtung zur Verabreichung des injizierbaren Produkts bilden. Die einzelnen Komponenten des Systems bilden einen Bausatz für eine Verabreichungsvorrichtung. Durch die Verwendung von Fertigbauteilen ist es leicht auf besondere Anforderungen anzugehen, in dem nur einzelne Bauteile verändert werden müssen. Beispielsweise kann dieselbe Antriebseinheit für verschiedenartig ausgebildete fertige Injektionsspritzen verwendet werden oder es können für eine bestimmte Injektionsspritze unterschiedliche Antriebseinheiten herangezogen werden.

Bei einer bevorzugten Ausführungsform des Verabreichungssystems nach der vorliegenden Erfindung ist eine Rückzugsvorrichtung zum Zurückziehen der Injektionsnadel ins Innere des Gehäuses vorgesehen. Bei einer besonders bevorzugten Ausführungsform ist die Rückzugsvorrichtung in der Injektionsspritze angeordnet und zieht die Injektionsnadel ins Innere des Spritzengehäuses zurück. Mit der Rückzugsvorrichtung wird die Injektionsnadel nach einer Verabreichung eines injizierbaren Produkts aus dem Gewebe herausgezogen und direkt wieder in dem Gehäuse untergebracht. Die Injektionsnadel ist demnach für einen Anwender zu keinem Zeitpunkt während der Verabreichung zugänglich, wodurch eine Verletzungsgefahr unterbunden wird. Eine Injektionsspritze mit einer solchen Rückzugsvorrichtung kann als Massenprodukt in großer Stückzahl hergestellt werden. Eine derartige Spritze ist z. B. von NMT New Medical Technology erhältlich.

Es ist vorteilhaft, wenn die Antriebseinheit neben dem Antrieb für die Injektionsspritze auch einen Antrieb für das Vorschubglied der Injektionsspritze bildet, durch den das Vorschubglied relativ zu dem Spritzengehäuse für eine Verabreichung des injizierbaren Produkts vorgeschoben wird. Als Vorschubglied kann dabei eine einfache Kolbenstange dienen, die den Stopfen innerhalb einer Produktkammer der Injektionsspritze vorschiebt, wie es herkömmlicherweise von Hand durchgeführt wird. Hierfür kann die Antriebseinheit ein Antriebsglied aufweisen, das für eine Verabreichung in einem ersten Schritt die Injektionsspritze relativ zum Gehäuse des Verabreichungssystem und in einem zweiten Schritt das Vorschubglied der Injektionsspritze relativ zu dem Spritzengehäuse antreibt. Daraus ergibt sich ein automatisches Einstechen der Injektionsnadel und darauffolgendes Ausschütten des Produkts aus der Injektionsspritze.

Bei einer besonders vorteihaften Ausführungsform wird die Rückzugsvorrichtung von der Antriebseinheit ausgelöst. Das Auslösen der Rückzugsvorrichtung kann z. B. durch einen Anschlag des Vorschubglieds nach einer erfolgten Verabreichung des injizierbaren Produkts aus der Injektionsspritze erfolgen. Das Vorschubglied kann z. B. am Ende des Vorschubs durch die Produktkammer gegen einen Anschlag, der z. B. einen Abschluss der Kammer bildet, stoßen und damit die Rückzugsvorrichtung auslösen. Wird das Vorschubglied mit Hilfe der Antriebseinheit in Vorschubrichtung bewegt, kann die Rückzugsvorrichtung mittels der Antriebseinheit ausgelöst werden. Bei dieser Ausführungsform des erfindungsgemäßen Verabreichungssystem erfolgt mit Hilfe der Antriebseinheit automatisch ein Vorschub der Injektionsspritze zum Einstechen der Injektionsnadel, ein Vorschub des Vorschubglieds zur Ausschüttung des Produkts aus der Injektionsspritze und ein Rückzug der Injektionsnadel in das Gehäuse. Die Injektionsnadel ist dabei zu jedem Zeitpunkt während der Verabreichung unzugänglich.

Das Verabreichungssystem nach der vorliegenden Erfindung kann eine Auslöseeinrichtung aufweisen, welche die Antriebseinheit und vorzugsweise auch die Rückzugsvorrichtung auslöst. Hierfür werden die Antriebseinrichtung und die Rückzugsvorrichtung in einem vorgespannten Zustand gehalten, der durch Betätigung der Auslöseeinrichtung gelöst werden kann, so dass die Antriebseinheit und die Rückzugsvorrichtung durch die Vorspannkraft ihre Funktion erfüllen können. Die Auslöseeinrichtung ist z. B. derart ausgebildet, dass die Antriebseinrichtung mittels einer Feder, die z. B. zwischen dem Antriebsglied und einem Gehäuseteil vorgesehen ist, vorgespannt wird und in dem vorgespannten Zustand gegenüber dem Gehäuseteil fixiert wird. Zum Auslösen wird das Antriebsglied von der Auslöseeinrichtung gegenüber dem Gehäuse gelöst, so dass es sich relativ zu dem Gehäuseteil in Vorschubrichtung bewegt und dadurch das Vorschubglied der Injektionsspritze antreibt und letztlich die Rückzugsvorrichtung z. B. wie oben beschrieben auslöst.

Vorzugsweise wird die Auslöseeinrichtung durch zwei relativ zueinander bewegliche Gehäuseteile des Gehäuses gebildet. Ein erstes Gehäuseteil dient dazu, die Antriebseinheit in einem vorgespannten Zustand zu fixieren und das zweite Gehäuseteil dient zum Auslösen der Auslöseeinrichtung, d.h. zum Lösen des vorgespannten Zustands. Hierfür wird das zweite Gehäuseteil gegenüber dem ersten Gehäuseteil verschoben, bis die Antriebseinheit, bzw. das Antriebsglied, freigegeben ist. Besonders bevorzugt wird ein zweites dem Gewebe naheliegendes Gehäuseteil entgegen der Vorschubrichtung gegenüber einem ersten entfernt vom Gewebe liegenden Gehäuseteil verschoben. Wird das Verabreichungssystem z. B. an dem ersten Gehäuseteil gehalten und für eine Verabreichung auf eine Oberfläche eines Gewebes aufgesetzt, kann durch Aufdrücken des Verabreichungssystems das zweite Gehäuseteil gegenüber dem ersten Gehäuseteil entgegen der Vorschubrichtung verschoben werden und somit die Antriebseinheit ausgelöst werden. Dadurch ist kein gesonderter Handgriff zum Auslösen des Verabreichungssystems notwendig. Das System muss zur Verabreichung des injizierbaren Produkts lediglich auf eine Gewebeoberfläche aufgesetzt und angedrückt werden. Der vollständige Verabreichungsvorgang wird dann automatisch durch ein Zusammenwirken der Antriebseinheit und der Rückzugsvorrichtung durchgeführt.

Ein erfindungsgemäßes System zur Verabreichung eines injizierbaren Produkts wird anhand eines Ausführungsbeispiels mit Hilfe der Zeichnung näher erläutert. In dieser stellen dar:
- Figur 1: Längsschnitt durch Bauelemente eines Verabreichungssystems nach der vorliegenden Erfindung,
- Figur 2: Längsschnitt durch ein zusammengesetztes Verabreichungssystem nach der vorliegenden Erfindung in einer gesicherten Stellung,
- Figur 3: Längsschnitt durch das Verabreichungssystem nach Figur 2 in einer ausgelösten Stellung,
- Figur 4: Längsschnitt durch das Verabereichungssystem nach Figur 2 in einer eingestochenen Stellung,
- Figur 5: Längsschnitt durch das Verabreichungssystem nach Figur 2 in einer ausgeschütteten Stellung und
- Figur 6: Längsschnitt durch das Verabreichungssystem nach Figur 2 in einer Nadelrückzugstellung.

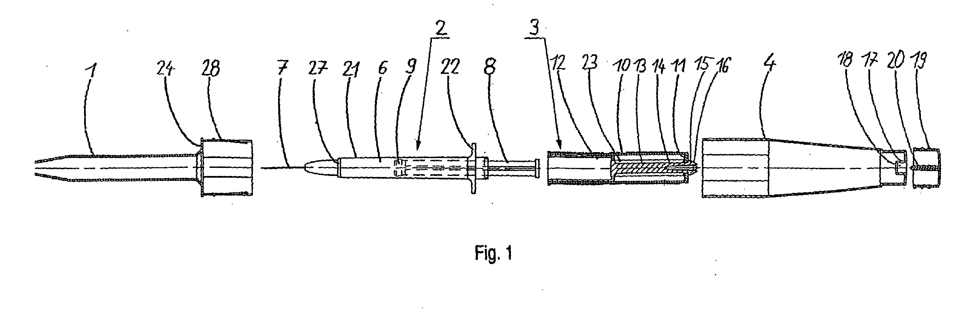

In Figur 1 sind die einzelnen Bauteile eines erfindungsgemäßen Verabreichungssystems gezeigt. Das Verabreichungssystem umfasst ein erstes vorderes Gehäuseteil 1, eine Injektionsnadel 2, eine Antriebseinheit 3 und ein zweites hinteres Gehäuseteil 4. Das erste Gehäuseteil 1 weist einen hülsenförmig ausgebildeten vorderen Bereich mit schmalem Durchmesser zur Aufnahme der Injektionsspritze 2 auf. Der hintere Bereich des ersten Gehäuseteils 1 ist gabelförmig ausgebildet. Am Ende des hülsenförmigen vorderen Bereichs stehen senkrecht zur Umfangsfläche zwei sich gegenüberliegende Stege ab, von welchen in Längsrichtung des hülsenförmigen Bereichs zwei Arme 28 abstehen. Der Abstand der sich gegenüberliegenden Arme 28 zueinander ist größer als der Durchmesser des hülsenförmigen Bereichs. Der hintere gabelförmige Bereich des ersten Gehäuseteils 1 dient als Halterung oder Anschlag für die Injektionsspritze 2. Als Injektionsspritze wird eine herkömmliche Spritze verwendet, wie sie z. B. von NMT New Medical Technology hergestellt wird. Die Injektionsspritze weist ein Spritzengehäuse 21 mit einer darin untergebrachten Kammer 6 für das fluide Produkt und eine Injektionsnadel 7 am vorderen Ende auf. Am hinteren Ende ragt ein Vorschubglied in Form einer Kolbenstange 8 aus der Injektionsspritze 2 hervor, das relativ zur Spritze in Längsrichtung verschiebbar ist und zum Vorschieben eines Stopfens 9 innerhalb der Kammer 6 dient. Am hinteren Ende des Spritzengehäuses 21 ist eine in Umfangsrichtung des Gehäuses 21 abstehende Manschette 22 angebracht.

Die Antriebseinheit 3 weist eine Hülse 10 auf, die am vorderen Ende offen und am hinteren Ende mit einer Abflusswand 11 mit einer zentralen Öffnung versehen ist. In der Hülse 10 ist ein Antriebsglied 12 gelagert, das relativ zur Hülse 10 in Längsrichtung verschiebbar ist. Das Antriebsglied 12 ist in einem vorderen Bereich hülsenförmig ausgebildet, wobei die Außenumfangsfläche des Hülsenbereichs an der Innenumfangsfläche der Hülse 10 anliegt. An den vorderen hülsenförmigen Bereich schließt sich eine Stange 13 an, die zentral entlang der Längsachse der Hülse 10 verläuft. Am hinteren Ende spaltet sich die Spange 13 in zwei sich gegenüberliegende Flügel 14, die an ihren Enden von der Umfangsfläche der Stange 13 nach außen abragende Vorsprünge 15 und spitz auf die Längsachse zulaufende schräge Abschlussflächen 16 aufweisen. Zwischen den sich gegenüberliegenden Flügeln 14 ist ein Spalt ausgebildet, so dass die Flügel 14 durch Aufwenden einer Kraft radial nach innen, d.h. zusammengebogen werden können. Das Ende der Stange 13 des Antriebsgliedes 12 ragt durch die zentrale Öffnung der Abschlusswand 11 hindurch und die Vorsprünge 15 kommen an der Außenseite der Abschlusswand 11 zu liegen. In dieser Stellung ist das Antriebsglied 12 gegenüber der Hülse 10 nicht in Vorschubrichtung verschiebbar. Das Antriebsglied 12 wird von einer Feder 23 in vorgespanntem Zustand gehalten, indem die Vorsprünge 15 an der Abschlusswand 11 anliegen. Hierfür ist die Feder 23 zwischen dem hülsenartigen Bereich des Antriebsglieds 12 und der Innenfläche der Abschlusswand 11 der Hülse 10 um die Stange 13 herum eingesetzt.

Das zweite hintere Gehäuseteil 4 ist derart hülsenförmig ausgebildet, dass es die Antriebseinheit aufnehmen kann und sich mit seinem vorderen Ende an den Bereich mit breitem Durchmesser des ersten Gehäuseteils 1 formschlüssig anschließt. Das hintere Ende weist einen nach innen gerichteten koaxial angeordneten hülsenartigen Fortsatz 17 auf, der um eine zentrale Öffnung 18 angeordnet ist. Auf das hintere Ende des zweiten Gehäuseteils 4 ist eine Sicherungskappe 19 formschlüssig aufsetzbar. Die Sicherungskappe 19 weist einen nach innen in Richtung des zweiten Gehäuseteils 4 gerichteten Dorn 20 auf. In auf das Gehäuseteil 4 aufgesetztem Zustand ragt der Dorn 20 durch die Öffnung 18 des Gehäuseteils 4 hindurch. Wie in Figur 1 zu sehen ist, ist das System zur Verabreichung eines injizierbaren Produkts nach der Erfindung als eine Art Baukastensystem aufgebaut.

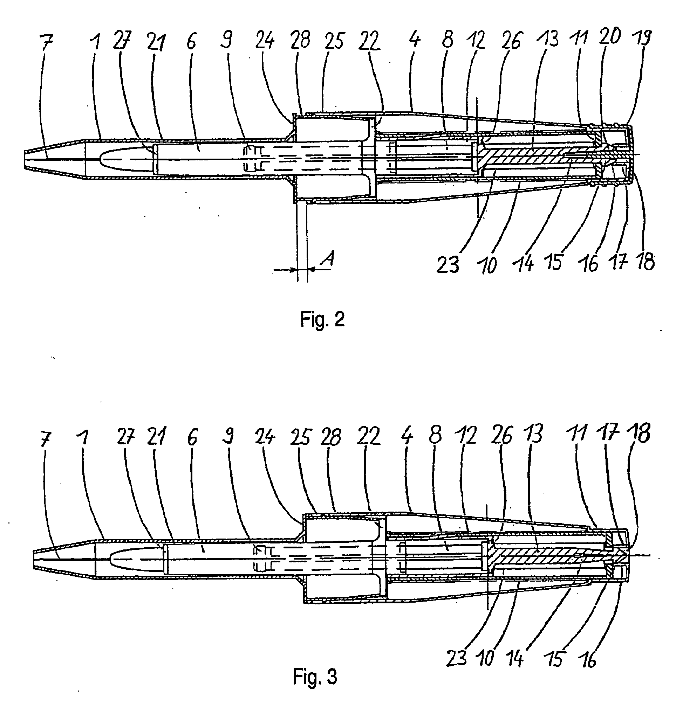

In Figur 2 ist das in Figur 1 in Einzelteilen gezeigte Verabreichungssystem in zusammengesetztem Zustand dargestellt. Die Injektionsspritze 2 ist mit der Injektionsnadel 7 im Inneren des vorderen Bereichs mit schmalem Durchmesser des ersten Gehäuseteils 1 untergebracht. Die Injektionsnadel 7 wird dabei von dem ersten Gehäuseteil 1 umgeben. Die Injektionsspritze 2 wird so weit in Vorschubrichtung in das erste Gehäuseteil 1 eingeführt, bis die Manschette 22 gegen das Ende der Arme 28 und des gabelförmigen Bereichs des ersten Gehäuseteils 1 stößt. Die Arme 28 sind an ihrem Ende geringfügig nach innen gebogen und weisen an ihren Enden Haltevorkehrungen zur Aufnahme des Randes der Manschette 22 auf. In dieser gesicherten Stellung kann die Injektionsspritze 2 gegenüber dem ersten Gehäuseteil 1 nicht weiter in Vorschubrichtung bewegt werden. Die nach hinten aus der Injektionsspritze 2 herausragende Kolbenstange 8 ragt ins Innere des hülsenförmigen vorderen Bereichs des Antriebsglieds 12 und stößt an die Stirnseite der Stange 13. In diesem Zustand stößt das vordere Ende der Hülse 10 und das vordere Ende des Antriebsglieds 12 gegen die Manschette 22 des Spritzengehäuses 21. Das zweite hintere Gehäuseteil 4 wird über die Antriebseinheit 3 und über den hinteren Teil der Injektionsspritze 2 auf den gabelförmigen Bereich des ersten Gehäuseteils 1 formschlüssig aufgesetzt.

Auf das hintere Ende des zweiten Gehäuseteils 4 ist die Sicherungskappe 19 aufgesetzt. Dabei ragt der Dorn 20 der Sicherungskappe 19 durch die zentrale Öffnung 18 des zweiten Gehäuseteils 4 und in den Spalt zwischen den Flügeln 14 der Stange 13 des Antriebsglieds 12. Durch den Dorn 20 der Sicherungskappe 19 werden die Flügel daran gehindert, sich nach innen zu biegen. Die vorderen Enden des hülsenförmigen Fortsatzes 17 stoßen an die schrägen Abschlussflächen 16 der Stange 13.

Das vordere Ende des zweiten Gehäuseteils 4, das auf den gabelförmigen Bereich des ersten Gehäuseteils 1 aufgeschoben ist, endet mit einem Abstand A vor den Stegen 24. Die nach außen gerichteten Stege 24 an dem ersten Gehäuseteil 1 bilden einen Anschlag für das zweite Gehäuseteil 4, der bei einem Verschieben der Gehäuseteile 1 und 4 gegeneinander ein Vorschieben des zweiten Gehäuseteils 4 über die Stege 24 hinaus in Vorschubrichtung verhindert.

In der in Figur 2 gezeigten Stellung wird ein Vorschieben des zweiten Gehäuseteils 4 gegenüber dem ersten Gehäuseteil 1 verhindert, indem das vordere Ende des hülsenartigen Fortsatzes 17 gegen die schrägen Abschlussflächen 16 am hinteren Ende der Stange 13, die Vorsprünge 15 am Antriebsglied 12 gegen die Außenfläche der Abschlusswand 11 der Hülse 10, das vordere Ende der Hülse 10 gegen die Manschette 22 der Injektionsspritze 2 und die Manschette 22 gegen die Arme 28 des ersten Gehäuseteils 1 abgestützt wird. Daraus ergibt sich insgesamt eine zueinander feststehende Anordnung der einzelnen Bauelemente des Verabreichungssystems. Es kann zusätzlich eine Rasteinrichtung, bzw. eine Führungsrille 25 vorgesehen sein, die ein Auseinanderziehen des ersten Gehäuseteils 1 und des zweiten Gehäuseteils 4 durch ein Abziehen des zweiten Gehäuseteils 4 entgegen der Vorschubrichtung gegenüber dem ersten Gehäuseteil 1 erschwert oder verhindert. In diese Stellung befindet sich das Verabreichungssystem in einer gesicherten Stellung, da durch die aufgesetzte Sicherungskappe 19 ein Zusammendrücken der Flügel 14 und damit eine Bewegung der einzelnen Elemente zueinander verhindert wird.

Der nach innen ragende hülsenartige Fortsatz 17, die Flügel 14 mit den schrägen Abschlussflächen 16 und den Vorsprüngen 15 und die zentrale Öffnung in der Abschlusswand 11 der Antriebseinheit 3 und die vorgespannte Feder 23 bilden eine Auslöseeinrichtung, durch die die Antriebseinheit 3 und die Rückzugsvorrichtung der Injektionsspritze 2 ausgelöst werden können.

In Figur 3 ist das Verabreichungssystem in einer Auslösestellung gezeigt. Die Sicherungskappe 19 ist von dem zweiten Gehäuseteil 4 abgenommen, womit der Dorn 20 aus dem Spalt zwischen den Flügeln 14 der Stange 13 entfernt wird. Das zweite Gehäuseteil 4 ist aus der gesicherten Stellung gegenüber dem ersten Gehäuseteil 1 um den Abstand A in Vorschubrichtung vorgeschoben und stößt mit dem vorderen Ende an die Stege 24. Bei der Vorschubbewegung des zweiten Gehäuseteils 4 gegenüber dem ersten Gehäuseteil 1 wird das zweite Gehäuseteil 4 auch gegenüber der Injektionsspritze 2 und der Antriebseinheit 3 in Vorschubrichtung verschoben. Dabei wird das vordere Ende des nach innen ragenden hülsenartigen Fortsatzes 17 derart über die schrägen Abschlussflächen 16 der Flügel 14 geschoben, dass die Flügel 14 nach innen zusammengedrückt werden. Dabei verringert sich der Durchmesser der Außenkanten der Vorsprünge 15 an den Flügeln 14 soweit, dass die Vorsprünge 15 nicht mehr an der Abschlusswand 11 der Hülse 10 anliegen, sondern durch die zentrale Öffnung in der Abschlusswand 11 passen. Das Antriebsglied 12 ist daher gegenüber der Hülse 10 der Antriebseinheit 3 in Vorschubrichtung verschiebbar.

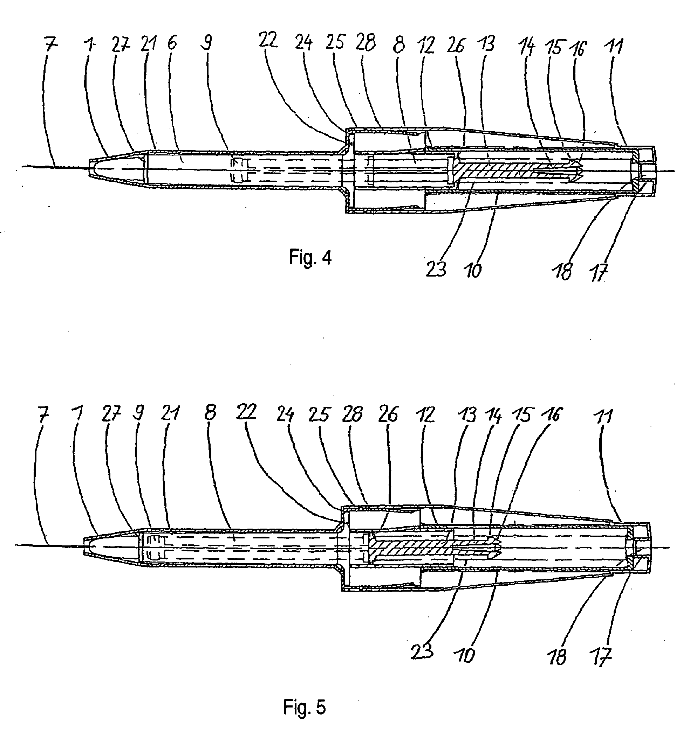

In Figur 4 ist das Verabreichungssystem in einer eingestochenen Stellung gezeigt, in der die Injektionsnadel 7 nach vorne aus dem ersten Gehäuseteil 1 herausragt. Durch die Freigabe des Antriebsglieds 12 wird dieses durch die Vorspannung der Feder 23 gegenüber der Hülse 10 der Antriebseinheit 3 verschoben. Da das vordere Ende des hülsenförmigen Bereichs des Antriebsglieds 12 an der Manschette 22 der Injektionsspritze 2 anliegt, wird die Antriebskraft der Feder 23 auf die Manschette 22 übertragen. Die Manschette 22 kann derart an den Enden der Arme 28 aufsitzen, dass sie bei Einwirken einer Kraft von den Armen 28 abrutschen und sich zwischen den Armen 28 hindurch bewegen kann. Durch den Vorschub des Antriebsglieds 12 wird die Manschette 22 daher aus ihrem Anschlag an den Armen 28 des ersten Gehäuses 1 gelöst und gegenüber dem ersten Gehäuse 1 verschoben, bis sie an den Stegen 24 anschlägt. Die Injektionsspritze 2 wird daher durch die Vorschubbewegung des Antriebsglieds 12 gegenüber dem ersten Gehäuseteil 1 in Vorschubrichtung geschoben und die Injektionsnadel 7 tritt aus dem vorderen Ende des Gehäuseteils 1 hervor.

Vorzugsweise ist zum Lösen des Anschlags der Manschette 22 an den Armen 28 am zweiten Gehäuseteil 4 eine Steuerkurve vorgesehen. Bei der Verschiebung des ersten Gehäuseteils 1 und des zweiten Gehäuseteils 4 gegen einander, können die Arme 28 mit Hilfe der Steuerkurve aufgeweitet werden, so dass die Manschette 22 in Vorschubrichtung gegenüber dem ersten und zweiten Gehäuseteil verschoben werden kann. Die Steuerkurve kann z. B. durch eine Oberflächenstruktur z. an der Innenumfangsfläche des zweiten Gehäuseteils 4 gegeben sein. Die Aussenseiten der Arme 28 können z. B. über ein Kontaktelement oder direkt an der Steuerkurve anliegen und durch den Kurvenverlauf aus einer nach innen gerichteten Vorspannung frei gegeben werden.

In Figur 5 ist das Verabreichungssystem in einer ausgeschütteten Stellung gezeigt, in der die Kolbenstange 8 der Injektionsspritze 2 gegenüber dem Spritzengehäuse 21 in Vorschubrichtung vorgeschoben ist und den Stopfen 9 in der Kammer 6 zur Ausschüttung des fluiden Produkts vorgeschoben hat. An dem Antriebsglied 12 der Antriebseinheit 3 ist im Übergang zwischen dem hülsenartigen vorderen Bereich zu der Stange 13 eine Sollbruchstelle 26 vorgesehen. Durch die Vorschubkraft der Feder 23 und den Anschlag der Manschette 22 an den Stegen 24 des ersten Gehäuseteils 1 beim Vorschub der Injektionsspritze 2 bricht die Sollbruchstelle 26 durch die plötzliche Verzögerung der Spritze beim Anschlag. Die Stange 13 wird durch die Vorschubkraft der Feder 23 innerhalb des hülsenartigen Bereichs des Antriebsglieds 12 in Vorschubrichtung weiter vorgeschoben und treibt dadurch die Kolbenstange 8 ebenfalls in Vorschubrichtung innerhalb dem Spritzengehäuse 21 an. Dadurch wird der Stopfen 9 innerhalb der Kammer 6 in Vorschubrichtung bewegt und das fluide Produkt über die Injektionsnadel 7 aus der Kammer 6 ausgeschüttet.

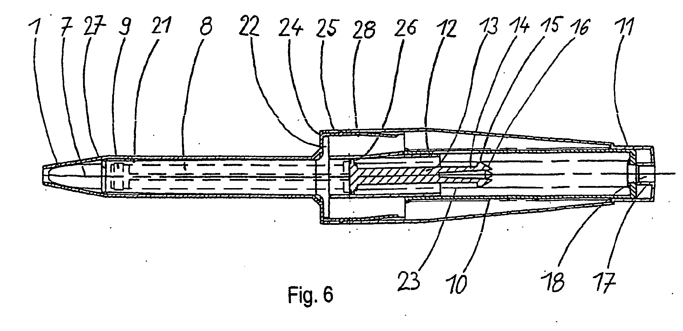

In Figur 6 ist das Verabreichungssystem in einer Nadelrückzugsstellung gezeigt, in der die Injektionsnadel 7 in das Innere des ersten Gehäuseteils 1 zurückgezogen ist. Die Injektionsspritze 2 ist mit einer Rückzugsvorrichtung zum Zurückziehen der Injektionsnadel versehen. Die Rückzugsvorrichtung kann z. B. dadurch betätigt werden, dass der von der Kolbenstange 8 vorgeschobene Stopfen 9 am Ende des Vorschubs gegen einen Auslöser 27 anschlägt, so dass die Injektionsnadel 7 automatisch ins Innere des Spritzengehäuses 21 und damit ins Innere des ersten Gehäuseteils 1 zurückgezogen wird.

Der Verabreichungsvorgang mit dem erfindungsgemäßen Verabreichungssystem ist damit abgeschlossen. Der Verabreichungsvorgang erfolgt durch ein Verschieben des zweiten Gehäuseteils 4 gegenüber dem ersten Gehäuseteil 1, wobei das vordere Ende des ersten Gehäuseteils 1 auf einer Gewebeoberfläche an einer Injektionsstelle aufgesetzt wird, das Verabreichungssystem an dem zweiten Gehäuseteil 4 gehalten und an diesem auf die Gewebeoberfläche aufgedrückt wird. Durch die Verschiebung wird die Antriebseinheit und die Rückzugsvorrichtung ausgelöst. Das heißt zunächst wird die Injektionsnadel 7 in das Gewebe eingestochen, anschließend die Kolbenstange 8 zur Ausschüttung vorgeschoben und letztlich die Injektionsnadel 7 in das Spritzengehäuse 21 zurückgezogen. Während des gesamten Vorgangs ist die Injektionsnadel 7 vor Zugang geschüttet. Nach der Verabreichung des injizierbaren Produkts können das erste Gehäuseteil 1 und das zweite Gehäuseteil 4 voneinander getrennt und die Injektionsspritze 2 entnommen werden. Es kann eine neue Injektionsspritze 2 mit den übrigen Bauelementen des Verabreichungssystems zusammengesetzt werden, so dass das System für eine erneute Verabreichung bereit steht. Es ist jedoch auch möglich, die einzelnen Bauteile des Verabreichungssystems als Wegwerfteile auszubilden.

Das erfindungsgemäße Verabreichungssystem wurde anhand eines Ausführungsbeispiels verdeutlicht. Grundsätzlich ist es jedoch möglich das Baukastenprinzip des Systems auch mit anderen Bauteilen zu verwirklichen. Zum Beispiel können verschiedenartige Injektionsspritzen verwendet werden oder verschiedene Antriebseinheiten, die vorteilhafterweise in bereits vorgespanntem Zustand vorliegen.

### Bezugszeichen:

- 1: erstes Gehäuseteil
- 2: Injektionsspritze
- 3: Antriebseinheit
- 4: zweites Gehäuseteil
- 5: -
- 6: Kammer
- 7: Injektionsnadel
- 8: Kolbenstange
- 9: Stopfen
- 10: Hülse
- 11: Abschlusswand
- 12: Antriebsglied
- 13: Stange
- 14: Flügel
- 15: Vorsprung
- 16: Abschlussfläche
- 17: Fortsatz
- 18: Öffnung
- 19: Sicherungskappe
- 20: Dorn
- 21: Spritzengehäuse
- 22: Manschette
- 23: Feder
- 24: Steg
- 25: Führungsrille
- 26: Sollbruchstelle
- 27: Auslöser
- 28: Arm
- A: Abstand

## Patentansprüche

1. System zur Verabreichung eines injizierbaren Produkts umfassend:
a) eine austauschbare Injektionsspritze (2), aus der das injizierbare Produkt durch eine Injektionsnadel (7) verabreicht wird,
b) eine Antriebseinheit (3) und
c) ein Gehäuse, das ein erstes Gehäuseteil (1) zur Aufnahme der Injektionsspritze (2) und ein zweites Gehäuseteil (4) zur Aufnahme der Antriebseinheit (3) aufweist, wobei das erste Gehäuseteil (1) mit dem zweiten Gehäuseteil (4) zusammengesetzt werden kann,
**dadurch gekennzeichnet, dass**
d) das erste Gehäuseteil (1) einen gabelförmigen Bereich aufweist, der als Halterung oder Anschlag für die Injektionsspritze (2) derart dient, dass die Injektionsspritze (2) gegenüber dem Gehäuseteil (1) nicht weiter in Vorschubrichtung bewegt werden kann, so dass die Injektionsspritze (2) in einer gesicherten Stellung ist, in der die Injektionsnadel (7) im Inneren des ersten Gehäuseteils (1) untergebracht ist,
e) wobei die Antriebseinheit (3) die Injektionsspritze (2) in Längsrichtung des Gehäuses (1, 4) relativ zum Gehäuse (1, 4) derart antreibt, dass die Injektionsspritze (2) aus ihrem Anschlag oder ihrer Halterung gelöst und in eine eingestochene Stellung vorgeschoben wird, in der die Injektionsnadel (7) aus dem ersten Gehäuseteil (1) herausragt.

2. Verabreichungssystem nach Anspruch 1, wobei die Injektionsspritze (2), die Antriebseinheit (3) und das Gehäuse (1, 4) als getrennte Bauteile vorliegen.

3. Verabreichungssystem nach einem der vorhergehenden Ansprüche, **dadurch gekennzeichnet, dass** der gabelförmige Bereich des ersten Gehäuseteils (1) zwei Arme (28) umfasst und die Injektionsspritze (2) in einer gesicherten Stellung gegen ein Ende der Arme (28) stösst, wobei sich die Arme (28) zum Lösen der Injektionsspritze (2) aufweiten.

4. Verabreichungssystem nach einem der vorhergehenden Ansprüche, **dadurch gekennzeichnet, dass** am zweiten Gehäuseteil (4) eine Steuerkurve vorgesehen ist, durch welche die Arme (28) aufgeweitet werden.

5. Verabreichungssystem nach dem vorherigen Anspruch, **dadurch gekennzeichnet, dass** die Arme (28) über ein Kontaktelement oder direkt an der Steuerkurve anliegen und durch den Kurvenverlauf freigegeben werden.

6. Verabreichungssystem nach einem der vorhergehenden Ansprüche, **dadurch gekennzeichnet, dass** die Injektionsspritze (2) aus dem ersten Gehäuseteil (1) entnehmbar ist und eine neue Injektionsspritze (2) einsetzbar ist.

7. Verabreichungssystem nach einem der vorhergehenden Ansprüche, wobei eine Rückzugsvorrichtung zum Zurückziehen der Injektionsnadel (7) ins Innere des ersten Gehäuseteils (1) vorgesehen ist.

8. Verabreichungssystem nach dem vorhergehenden Anspruch, wobei die Rückzugsvorrichtung die Injektionsnadel (7) ins Innere eines Spritzengehäuses (21) zurückzieht.

9. Verabreichungssystem nach einem der vorhergehenden Ansprüche, wobei die Antriebseinheit (3) an einem Spritzengehäuse (21) zum Vorschieben der Injektionsspritze (2) gegenüber dem Gehäuse (1, 4) angreift und ein Vorschubglied (8) der Injektionsspritze (2) relativ zu einem Spritzengehäuse (21) zur Verabreichung des injizierbaren Produkts antreibt.

10. Verabreichungssystem nach einem der vorhergehenden Ansprüche 7 oder 8, wobei die Antriebseinheit (3) die Rückzugsvorrichtung auslöst.

11. Verabreichungssystem nach einem der vorhergehenden Ansprüche, wobei eine Auslöseeinrichtung durch die zwei relativ zueinander beweglichen Gehäuseteile (1, 4) gebildet wird.

## Claims

1. System for administering an injectable product, comprising:
a) a replaceable injection syringe (2) from which the injectable product is administered by an injection needle (7),
b) a drive unit (3) and
c) a housing which has a first housing part (1) to house the injection syringe (2) and a second housing part (4) to house the drive unit (3), wherein the first housing part (1) can be combined with the second housing part (4),
**characterized in that**
d) the first housing part (1) has a forked zone which serves as holder or stop for the injection syringe (2) such that the injection syringe (2) cannot be moved further in the direction of advance vis-à-vis the housing part (1), with the result that the injection syringe (2) is in a secured position in which the injection needle (7) is accommodated inside the first housing part (1),
e) wherein the drive unit (3) drives the injection syringe (2) in longitudinal direction of the housing (1, 4) relative to the housing (1, 4) such that the injection syringe (2) is detached from its stop or its holder and is advanced into an inserted position in which the injection needle (7) projects from the first housing part (1).

2. Administration system according to claim 1, wherein the injection syringe (2), the drive unit (3) and the housing (1, 4) are separate components.

3. Administration system according to one of the previous claims, **characterized in that** the forked zone of the first housing part (1) comprises two arms (28) and the injection syringe (2) strikes in a secured position against one end of the arms (28), wherein the arms (28) widen to release the injection syringe (2).

4. Administration system according to one of the previous claims, **characterized in that** a radial cam at the second housing part (4) through which the arms (28) are widened is provided.

5. Administration system according to the previous claim, **characterized in that** the arms (28) are attached to the radial cam via a contact element or directly and are released by the course of the cam.

6. Administration system according to one of the previous claims, **characterized in that** the injection syringe (2) can be removed from the first housing part (1) and a new injection syringe (2) can be inserted.

7. Administration system according to one of the previous claims, wherein a withdrawal device for pulling the injection needle (7) back inside the first housing part (1) is provided.

8. Administration system according to the previous claim, wherein the withdrawal device draws the injection needle (7) back inside a syringe housing (21).

9. Administration device according to one of the previous claims, wherein the drive unit (3) engages with a syringe housing (21) to advance the injection syringe (2) vis-a-vis the housing (1, 4) and drives a feed member (8) of the injection syringe (2) relative to a syringe housing (21) to administer the injectable product.

10. Administration system according to one of claims 7 or 8, wherein the drive unit (3) triggers the withdrawal device.

11. Administration system according to one of the previous claims, wherein a triggering device is formed by the two housing parts (1, 4) movable relative to each other.

## Revendications

1. Système pour administrer un produit injectable comprenant :
a) une seringue d'injection échangeable (2) à partir de laquelle le produit injectable est administré par le biais d'une aiguille d'injection (7),
b) une unité d'entraînement (3) et
c) un boîtier qui comporte une première partie de boîtier (1) pour loger la seringue d'injection (2) et une deuxième partie de boîtier (4) pour loger l'unité d'entraînement (3), la première partie de boîtier (1) pouvant être assemblée avec la deuxième partie de boîtier (4), **caractérisé en ce que**
d) la première partie de boîtier (1) comporte une zone fourchue qui sert de support ou de butée à la seringue d'injection (2) de telle manière que la seringue d'injection (2) ne peut pas être déplacée plus vers l'avant par rapport à la partie de boîtier (1), si bien que la seringue d'injection (2) se trouve dans une position bloquée dans laquelle l'aiguille d'injection (7) est logée à l'intérieur de la première partie de boîtier (1),
e) l'unité d'entraînement (3) entraînant la seringue d'injection (2) dans le sens longitudinal du boîtier (1, 4) par rapport au boîtier (1, 4) de telle manière que la seringue d'injection (2) est détachée de sa butée ou de son support et avancée dans une position d'injection dans laquelle l'aiguille d'injection (7) dépasse de la première partie de boîtier (1).

2. Système d'administration selon la revendication 1, dans lequel la seringue d'injection (2), l'unité d'entraînement (3) et le boîtier (1, 4) sont disponibles en tant que composants séparés.

3. Système d'administration selon l'une des revendications précédentes, **caractérisé en ce que** la zone fourchue de la première partie de boîtier (1) comprend deux bras (28) et **en ce que** la seringue d'injection (2) se heurte dans une position bloquée à une extrémité des bras (28), les bras (28) s'écartant pour relâcher la seringue d'injection (2).

4. Système d'administration selon l'une quelconque des revendications précédentes, **caractérisé en ce qu'**une came de commande par laquelle les bras (28) sont élargis est prévue sur la deuxième partie de boîtier (4).

5. Système d'administration selon la revendication précédente, **caractérisé en ce que** les bras (28) sont en contact avec la came de commande via un élément de contact ou directement et sont libérés par le tracé de came.

6. Système d'administration selon l'une quelconque des revendications précédentes, **caractérisé en ce que** la seringue d'injection (2) peut être enlevée de la première partie de boîtier (1) et **en ce qu'**une nouvelle seringue d'injection (2) peut être mise en place.

7. Système d'administration selon l'une quelconque des revendications précédentes, dans lequel un dispositif de retour est prévu pour faire revenir l'aiguille d'injection (7) à l'intérieur de la première partie de boîtier (1).

8. Système d'administration selon la revendication précédente, dans lequel le dispositif de retour fait revenir l'aiguille d'injection (7) à l'intérieur d'un boîtier de seringue (21).

9. Système d'administration selon l'une quelconque des revendications précédentes, l'unité d'entraînement (3) agissant sur un boîtier de seringue (21) pour faire avancer la seringue d'injection (2) par rapport au boîtier (1, 4) et entraînant un élément d'avance (8) de la seringue d'injection (2) par rapport à un boîtier de seringue (21) pour administrer le produit injectable.

10. Système d'administration selon l'une des revendications précédentes,7 ou 8, dans lequel l'unité d'entraînement (3) déclenche le dispositif de retour.

11. Système d'administration selon l'une quelconque des revendications précédentes, un dispositif de déclenchement étant formé par les deux parties de boîtier (1, 4) mobiles l'une par rapport à l'autre.
